# EUROPEAN PATENT SPECIFICATION

(11) **EP 4 051 215 B1**
(45) Date of publication and mention of the grant of the patent: **02.12.2026**
(21) Application number: 20796822.3
(22) Date of filing: 29.10.2020
(51) Int. Cl.: A61K 8/04, A61K 8/73, A61Q 19/10

(54) **FOAM PRECURSOR LIQUID AND FOAM CLEANSING COMPOSITION**
SCHAUMVORLÄUFERFLÜSSIGKEIT UND SCHAUMREINIGUNGSZUSAMMENSETZUNG
LIQUIDE PRÉCURSEUR DE MOUSSE ET COMPOSITION DE MOUSSE DE NETTOYAGE

(30) Priority: 31.10.2019 EP 19206576
(43) Date of publication of application: 07.09.2022
(73) Proprietor: Unilever IP Holdings B.V., 6708 WH Wageningen (NL); Unilever Global IP Limited, Wirral, Merseyside CH62 4ZD (GB)
(72) Inventor: VASUDEVAN, Tirucherai Varahan, Trumbull, CT 06611 (US); DING, Zhaowei, Trumbull, CT 06611 (US)
(74) Representative: Unilever Patent Group
(86) International application number: PCT/EP2020/080395
(87) International publication number: WO 2021/084007

(56) References cited:
- WO-A1-2006/010440
- WO-A1-2018/114536
- WO-A2-2006/076065
- DE-A1- 102009 028 052
- PHARMA DOW ET AL: "Chemistry of METHOCEL(TM) Cellulose Ethers -A Technical Review", 1 January 2013 (2013-01-01), XP055687336, Retrieved from the Internet <URL:http://msdssearch.dow.com/PublishedLiteratureDOWCOM/dh_08e5/0901b803808e5f58.pdf?filepath=dowwolff/pdfs/noreg/198-02289.pdf&fromPage=GetDoc> [retrieved on 20200420]
- THE DOW CHEMICAL COMPANY: "Technical Handbook METHOCEL Cellulose Ethers", INTERNET CITATION, 1 September 2002 (2002-09-01), pages 1 - 29, XP002550462, Retrieved from the Internet <URL:http://www.dow.com/PublishedLiterature/dh_004f/0901b8038004fa1b.pdf?filepath=methocel/pdfs/noreg/192-01062.pdf&fromPage=GetDoc> [retrieved on 20091014]

## Description

### Field of the Invention

The present invention is directed to a liquid concentrate composition for a cleansing foam precursor liquid and a cleansing foam precursor liquid and a cleansing foam composition. More particularly, the invention is directed to a liquid concentrate composition and a cleansing foam precursor liquid that comprise a cellulose ether and surfactant. The precursor liquid results in a foam with excellent lather and foam characteristics upon dispensing from a pump foam dispenser, whereby simultaneously and unexpectedly, the liquid does not thicken to impede or clog the dispenser it is dispensed from.

Additionally, the foam precursor liquid of the present invention is pourable prior to dispensing, thereby making refilling and reusing pump foam dispensers very convenient for consumers conscious about using less plastic.

### Background of the Invention

Foaming cleansing products are often desired by consumers. Upon dispensing, they are easy to apply and spread, and they leave a good skin feel when rinsed off. Moreover, such products can deliver skin benefit agents, leave the skin feeling moist, and provide a nice format for delivering fragrances to the skin.

Unfortunately, it is often found that in order to produce a desirable foam that is dispensed from a pump actuator to yield a composition delivering consumer acceptable lathering, the precursor foam liquid needs to be thick. Thick precursor liquids invariably are difficult to pump, and characteristically result in a foam that impedes or even clogs the dispenser they are dispensed from. Thick foam precursor liquids can also be difficult to use in refill applications, making it undesirable for consumers to reuse plastic packaging.

Other foam generating products require foam boosting ingredients, like quaternary ammonium compounds. While these boosting ingredients work well at delivering a desirable foam, they often drive up the cost of the compositions, making diverse cleaning formats an unaffordable option for many consumers. Also, such quaternary compounds, in addition to expense, can make formulating difficult as they are traditionally incompatible with anionic surfactants.

It is of increasing interest to produce a foam precursor liquid that is easy to refill and a foam cleansing composition that results in good lather and foam characteristics, is pump foam dispenser "friendly" and is substantially free of quaternary ammonium compounds. The present invention, therefore, is directed to a precursor liquid and a concentrate composition for such precursor composition, and to a cleansing foam composition, that comprise a cellulose ether and surfactant. The precursor liquid results in a foam with excellent lather and foam characteristics upon dispensing from a dispenser, whereby simultaneously and unexpectedly, the foam does not thicken to impede or clog the pump foam dispenser it is dispensed from. Additionally, the foam precursor liquid of the present invention is pourable prior to dispensing, thereby making refilling and reusing pump foam dispensers very convenient for consumers conscious about using less plastic.

### Additional Information

Efforts have been described for making cleaning compositions. In U.S. Patent Application No. 2013/0210696 A1, cleaning compositions with squeakiness enhancers are disclosed.

Other efforts have been described for making cleaning compositions. In WO 2019/008937 A1, foam cleansers for cleaning keratinous substances are described.

Even other efforts have been described for making cleaning compositions. In U.S. Patent No. 9,320,697 B2, surfactant compositions used to prepare structural formulations are disclosed. Still other efforts have been described for making cleaning compositions. In U.S. Patent No.

5,696,069, personal foaming cleansing compositions with one or more surfactants is described.

DE 10 2009 028052 discloses a cosmetic cleansing composition containing a) 3 to 20% by weight of a surfactant base of at least one mild anionic surfactant and at least one mild amphoteric/zwitterionic surfactant, b) at least one non-ionic cellulose ether (claim 1), wherein the ratio of anionic surfactant(s) to the zwitterionic/amphoteric surfactant(s) is 3:1 to 1:2 , wherein the cleaning agent is characterized in that it contains 0.005 to 5% by weight of a non-ionic cellulose ether b) selected from the group of hydroxymethyl cellulose, hydroxyethyl cellulose, hydroxypropyl cellulose or hydroxypropyl methyl cellulose and mixtures of these ethers.

None of the additional information describes a foam precursor liquid and foam cleansing composition as described and claimed in the present application.

### Summary of the Invention

In a first aspect, the present invention is directed to a liquid concentrate for a cleansing foam precursor liquid comprising:
a) cellulose ether comprising hydroxypropyl methylcellulose, the hydroxypropyl methylcellulose having a degree of methoxy substitution (DS) from 1.77 to 1.94 and a hydroxypropyl molar substitution (MS) from 0.10 to 0.25;
b) from 5 to 70% by weight of anionic surfactant, and from 5 to 70% by weight amphoteric and/or zwitterionic surfactant, the anionic to amphoteric and/or zwitterionic surfactant at a weight ratio from 5:1 to 1:5; and
c) from 15 to 40% by weight water (preferably from 20 to 35, and most preferably, from 22 to 30% by weight water),
wherein the cellulose ether makes up from 0.25 to 5% by weight of the foam precursor liquid and when present at 2.0% by weight in water yields a solution having a viscosity from 0.5 to 250 cps.

In a second aspect, the present invention is directed to a cleansing foam precursor liquid filled in a pump foam dispenser, the composition comprising:
a) cellulose ether comprising hydroxypropyl methylcellulose, the hydroxypropyl methylcellulose having a degree of methoxy substitution (DS) from 1.77 to 1.94 and a hydroxypropyl molar substitution (MS) from 0.10 to 0.25;
b) from 2 to 35% by weight of anionic surfactant, and from 2 to 35% by weight amphoteric and/or zwitterionic surfactant, the anionic to amphoteric and/or zwitterionic surfactant at a weight ratio from 5:1 to 1:5; and
c) from 55 to 95.75 % by weight water,
wherein the cellulose ether makes up from 0.25 to 5% by weight of the foam precursor liquid and when present at 2.0% by weight in water yields a solution having a viscosity from 0.5 to 250 cps.

Degree of methoxy substitution, as used herein, is defined to mean the amount of substituent groups on the anhydroglucose units of cellulose, designated by the average number of methoxy substituent groups attached to the ring, a concept referred to as "degree of substitution" (D.S.). By way of illustration, if all three available positions on each unit are substituted, the D.S. is designated as 3. Molar substitution (MS) is defined to mean the number of moles of hydroxypropyl groups per mole of anhydroglucose.

Skin, as used herein, is meant to include skin on the arms (including underarms), face, feet, neck, chest, hands, legs, buttocks and scalp (including hair). Foam cleansing composition is a composition ready for topical application and to be wiped off, and preferably, washed off, with water. Such a composition can be a home care cleaning composition but is preferably a shampoo, make-up wash, facial wash or personal care liquid body wash. The foam cleansing composition may, optionally, comprise medicinal or therapeutic agents, but preferably, is a wash which is cosmetic and non-therapeutic. In one embodiment of the invention, the foam cleansing composition is a home care composition like a table top or toilet cleaning composition. In another embodiment, the foam cleansing composition is a shampoo composition. In still another embodiment, the end use composition is a personal wash composition. As hereinafter described, the foam cleansing composition of the present invention may optionally comprise skin benefit ingredients added thereto such as emollients, vitamins and/or derivatives thereof, resorcinols, retinoic acid precursors, colorants, moisturizers, sunscreens, mixtures thereof or the like. The skin benefit ingredients (or agents) may be water or oil soluble. If used, oil soluble skin benefit agents typically make up to 2.0% by weight of the foam precursor liquid and foam cleansing composition whereby water soluble skin benefit agents, when used, typically make up to 10% by weight of the liquid and composition of the present invention. The precursor foam liquid and foam cleansing composition typically have a pH from 4.5 to 10, and preferably, 5 to 9, and most preferably, 6 to 8.5. Liquid and composition, as referred to herein, means the foam precursor liquid and foam cleansing composition, respectively. Viscosity, unless noted otherwise, is taken at 25 °C with a Discovery HR-2 Rheometer using sand blasted plates with a 100 micron gap and a shear rate of 5-15 s⁻¹. Excellent lather and foam characteristics mean having a compression force of at least 205 mN. The viscosity of the cleansing foam precursor liquid preferably is from 25 to 3000 cps. Compression force means a foam's resistance to compression as measured with a TA XT Plus texture analyser having a TA-94 back extrusion fixture with settings at: test mode, compression; pre-test speed, 10mm/second; test speed, 0.5mm/second; post-test speed, 2mm/second; testing mode, distance; distance 4mm; hold time, 0.01 second, trigger type, auto; trigger force, 0.005kg; compression force (peak) in milli-Newtons (mN). In the absence of explicitly stating otherwise, all ranges described herein are meant to include all ranges subsumed therein. As used herein, "substantially free of" means less than 1.0% by weight. Concentrate is meant to be diluted with water to contain 55 to 95.75% by weight water to thereby yield foam precursor liquid. Preferably, the concentrate comprises, independently, 10 to 80%, and preferably, 15 to 75% of the anionic, and amphoteric and/or zwitterionic surfactants. In an embodiment of the invention, the concentrate is from 9 to 45%, and preferably, from 25 to 75% by weight total surfactant. The term comprising is meant to encompass the terms consisting essentially of and consisting of. For the avoidance of doubt, and for illustration, the foam cleansing composition of this invention comprising surfactant, water and cellulose ether is meant to include a composition consisting essentially of the same and a composition consisting of the same. Except in the operating comparative examples, or where otherwise explicitly indicated, all numbers in this description indicating amounts or ratios of materials or conditions and/or physical properties of materials and/or use are to be understood as modified by the word "about".

### Detailed Description of the Preferred Embodiments

As to the cellulose ether suitable for use in the present invention, the same comprise hydroxypropyl methylcellulose. In an embodiment of the invention, the cellulose ether consists essentially of hydroxypropyl methylcellulose. The hydroxypropyl methylcellulose typically has a degree of methoxy substitution (DS) from 1.77 to 1.94 and a hydroxypropyl molar substitution (MS) from 0.10 to 0.25. In an embodiment of the invention, the DS is from 1.78 to 1.93. In still another embodiment, the DS is from 1.79 to 1.92, including all ranges subsumed therein. The hydroxypropyl methylcellulose preferably has an MS from 0.11 to 0.24. In yet another embodiment, the MS of the hydroxypropyl methylcellulose is from 0.12 to 0.23, including all ranges subsumed therein. When present at 2.0% by weight in water, the cellulose ether used in the present invention yields a solution having a viscosity from 0.5 to 250 cps. In an embodiment of the invention, the viscosity of a 2.0% by weight solution is 5 to 100 cps, and preferably, 20 to 50 cps, including all ranges subsumed therein. Typically, the foam precursor liquid and foam cleansing composition comprise from 0.2 to 5%, and preferably, from 0.3 to 4%, and most preferably, from 0.35 to 3% by weight cellulose ether, including all ranges subsumed therein. Preferred cellulose ethers suitable for use in this invention are made commercially available by Dow Chemical under the names Methocel E and Methocel F. Such preferred cellulose ethers consist of hydroxypropyl methylcellulose having the above-identified MS and DS values. In still another preferred embodiment, the cellulose ethers used have 25 to 30% methoxy substitution and 4 to 9% hydroxypropyl substitution, including all ranges subsumed therein. In yet another embodiment of the invention, cellulose ether makes up from 0.35 to 0.8% by weight of the liquid and composition, including all ranges subsumed therein.

The cleansing foam precursor liquid and foam cleansing composition typically comprise from 2 to 35% by weight of anionic surfactant, and from 2 to 35% by weight amphoteric and/or zwitterionic surfactant at a weight ratio of anionic surfactant to amphoteric and/or zwitterionic surfactant from 5:1 to 1:5. In a preferred embodiment, the anionic and amphoteric surfactants are each independently present in the foam precursor liquid and foam cleansing composition from 3 to 30% by weight, and most preferably, 5 to 25% by weight of the precursor liquid and of the foam composition, as the case may be, including all ranges subsumed therein.

In yet another preferred embodiment, anionic surfactant, and amphoteric and/or zwitterionic surfactant each independently make up from 4 to 30%, and preferably, from 5 to 20% by weight of the precursor composition liquid and of the cleansing foam composition. In still another embodiment, the anionic surfactant, and amphoteric and/or zwitterionic surfactant are at a weight ratio of 4:1 to 1:4, preferably 3:1 to 1:3. In yet another embodiment, the weight ratio of such surfactants is 1:1.5 to 1.5 :1. In yet another embodiment of the invention, total surfactant in the precursor liquid and foam composition is from 3.0 to 16% by weight based. In still another embodiment, total surfactant in the precursor liquid and foam composition is from 4.5 to 14.5% by weight, including all ranges subsumed therein. In even another embodiment, total surfactant in the precursor liquid and foam composition is from 7 to 13% by weight of the liquid and composition, including all ranges subsumed therein.

As to the anionic surfactant present in the foam precursor liquid and foam cleansing composition of the present invention, the anionic surfactant used can include aliphatic sulfonates, such as a primary alkane (e.g., C₈-C₂₂) sulfonate, primary alkane (e.g., C₈-C₂₂) disulfonate, C₈-C₂₂ alkene sulfonate, C₈-C₂₂ hydroxyalkane sulfonate or alkyl glyceryl ether sulfonate (AGS); or aromatic sulfonates such as alkyl benzene sulfonate. The anionic may also be an alkyl sulfate (e.g., C₁₂-C₁₈ alkyl sulfate) or alkyl ether sulfate (including alkyl glyceryl ether sulfates). Among the alkyl ether sulfates are those having the formula:

RO(CH₂CH₂O)ₙSO₃M

wherein R is an alkyl or alkenyl having 8 to 18 carbons, preferably 12 to 18 carbons, n has an average value of at least 1.0, preferably less than 5, and most preferably 1 to 4, and M is a solubilizing cation such as sodium, potassium, ammonium or substituted ammonium.

The anionic may also be alkyl sulfosuccinates (including mono- and dialkyl, e.g., C₆-C₂₂ sulfosuccinates); alkyl and acyl taurates (often methyl taurates), alkyl and acyl sarcosinates, sulfoacetates, C₈-C₂₂ alkyl phosphates and phosphonates, alkyl phosphate esters and alkoxyl alkyl phosphate esters, acyl lactates, C₈-C₂₂ monoalkyl succinates and maleates, sulphoacetates, alkyl glucosides and acyl isethionates, and the like.

Sulfosuccinates may be monoalkyl sulfosuccinates having the formula:

R¹O₂CCH₂CH(SO₃M)CO₂M;

and amide-MEA sulfosuccinates of the formula:
R¹CONHCH₂CH₂O₂CCH₂CH(SO₃M)CO₂M wherein R¹ ranges from C₈-C₂₂ alkyl.

Sarcosinates are generally indicated by the formula:
R²CON(CH₃)CH₂CO₂M, wherein R² ranges from C₈-C₂₀ alkyl.

Taurates are generally identified by formula:

R³CONR⁴CH₂CH₂SO₃M

wherein R³ is a C₈-C₂₀ alkyl, R⁴ is a C₁-C₄ alkyl.

M is a solubilizing cation as previously described.

The foam precursor liquid and foam cleansing composition may contain C₈-C₁₈ acyl isethionates. These esters are prepared by a reaction between alkali metal isethionate with mixed aliphatic fatty acids having from 6 to 18 carbon atoms and an iodine value of less than 20. At least 75% of the mixed fatty acids have from 12 to 18 carbon atoms and up to 25% have from 6 to 10 carbon atoms.

The acyl isethionate may be an alkoxylated isethionate such as is described in Ilardi et al., U.S. Pat. No. 5,393,466, entitled "Fatty Acid Esters of Polyalkoxylated isethonic acid; issued Feb. 28, 1995; hereby incorporated by reference. This compound has the general formula:

R⁵C--O(O)--C(X)H--C(Y)H₂--(OCH--CH₂)ₘ--SO₃M

wherein R⁵ is an alkyl group having 8 to 18 carbons, m is an integer from 1 to 4, X and Y are each independently hydrogen or an alkyl group having 1 to 4 carbons and M is a solubilizing cation as previously described.

In an embodiment of the invention, the anionic surfactant used is sodium lauroyl glycinate, sodium cocoyl glycinate, sodium lauroyl glutamate, sodium cocoyl glutamate, sodium lauroyl isethionate, sodium cocoyl isethionate, sodium methyl lauroyl taurate, sodium methyl cocoyl taurate or a mixture thereof. Such anionic surfactants are commercially available from suppliers like Galaxy Surfactants, Clariant, Sino Lion and Innospec.

Amphoteric surfactants suitable for use in the invention (which depending on pH can be zwitterionic) include sodium acyl amphoacetates, sodium acyl amphopropionates, disodium acyl amphodiacetates and disodium acyl amphodipropionates where the acyl (i.e., alkanoyl group) can comprise a C₇-C₁₈ alkyl portion. Illustrative examples of the amphoteric surfactants suitable for use include sodium lauroamphoacetate, sodium cocoamphoacetate, sodium lauroamphoacetate, sodium cocoamphoacetate and mixtures thereof.

As to the zwitterionic surfactants employed in the present invention, such surfactants include at least one acid group. Such an acid group may be a carboxylic or a sulphonic acid group. They include often include quaternary nitrogen, and therefore, can be quaternary amino acids. They should generally include an alkyl or alkenyl group of 7 to 18 carbon atoms generally comply with an overall structural formula:
R⁶--[--C(O)--NH(CH₂)_{q}--]ᵣ--N⁺--(R⁷--)(R⁸)A--B where R⁷ is alkyl or alkenyl of 7 to 18 carbon atoms; R⁷ and R⁸ are each independently alkyl, hydroxyalkyl or carboxyalkyl of 1 to 3 carbon atoms; q is 2 to 4; r is 0 to 1; A is alkylene of 1 to 3 carbon atoms optionally substituted with hydroxyl, and B is --CO₂-- or --SO₃--.

Suitable zwitterionic surfactants for use in the present invention and within the above general formula include simple betaines of formula:

R⁶--N⁺--(R ⁷)(R⁸)CH₂CO₂⁻

and amido betaines of formula:
R⁶--CONH(CH₂)ₜ--N⁺--(R⁷XR⁸)CH₂CO₂⁻ where t is 2 or 3.

In both formulae R⁶, R⁷ and R⁸ are as defined previously. R⁶ may, in particular, be a mixture of C₁₂ and C₁₄ alkyl groups derived from coconut oil so that at least half, preferably at least three quarters of the groups R⁶ have 10 to 14 carbon atoms. R⁷ and R⁸ are preferably methyl.

A further possibility is that the zwitterionic surfactant is a sulphobetaine of formula:

R⁶--N⁺--(R⁷)(R⁸)(CH₂)₃SO₃⁻

or

R⁶--CONH(CH₂)ᵤ --N⁺--(R⁷)(R⁸)(CH₂)₃SO₃⁻

where u is 2 or 3, or variants of these in which --(CH₂)₃SO₃⁻ is replaced by-CH₂C(OH)(H)CH₂SO₃⁻.

In these formulae, R⁶, R⁷ and R⁸ are as previously defined.

Illustrative examples of the zwitterionic surfactants suitable for use include betaines like cocodimethyl carboxymethyl betaine, cocoamidopropyl betaine and laurylamidopropyl betaine. An additional zwitterionic surfactant suitable for use includes cocoamidopropyl sultaine. Such surfactants are made commercially available from suppliers like Stepan Company, and it is within the scope of the invention to employ mixtures of the aforementioned surfactants.

Nonionic surfactants may optionally be used in the foam precursor liquid and foam cleansing composition of the present invention. When used, nonionic surfactants are typically used at levels as low as 0.5, 1, 1.5 or 2% by weight and at levels as high as 6, 8, 10 or 12% by weight. The nonionics which may be used include in particular the reaction products of compounds having a hydrophobic group and a reactive hydrogen atom, for example aliphatic alcohols, acids, amides or alkylphenols with alkylene oxides, especially ethylene oxide either alone or with propylene oxide. Specific nonionic surfactant compounds are alkyl (C₆-C₂₂) phenols ethylene oxide condensates, the condensation products of aliphatic (C₈-C₁₈) primary or secondary linear or branched alcohols with ethylene oxide, and products made by condensation of ethylene oxide with the reaction products of propylene oxide and ethylenediamine. Other nonionic surfactants include long chain tertiary amine oxides, long chain tertiary phosphine oxides, dialkyl sulphoxides, and the like.

In an embodiment of the invention nonionic surfactants include fatty acid/alcohol ethoxylates having the following structures a) HOCH₂(CH₂)ₛ(CH₂CH₂O)ᵥ H or b) HOOC(CH₂)_{c}(CH₂CH₂O)_{d} H; where s and v are each independently an integer up to18; and c and d are each independently an integer from 1 or greater. In an embodiment of the invention, s and v are each independently 6 to 18; c and d are each independently 1 to 30. Other options for nonionic surfactants include those having the formula HOOC(CH₂)ᵢ-CH=CH-(CH₂)ₖ(CH₂CH₂O)_{z} H, where i, k are each independently 5 to 15; and z is 5 to 50. In another embodiment of the invention, i and k are each independently 6 to 12; and z is 15 to 35.

The nonionic may also include a sugar amide, such as a polysaccharide amide. Specifically, the surfactant may be one of the lactobionamides described in U.S. Pat. No. 5,389,279 to Au et al., entitled "Compositions Comprising Nonionic Glycolipid Surfactants issued Feb. 14, 1995; which is hereby incorporated by reference or it may be one of the sugar amides described in U.S. Pat. No. 5,009,814 to Kelkenberg, titled "Use of N-Poly Hydroxyalkyl Fatty Acid Amides as Thickening Agents for Liquid Aqueous Surfactant Systems" issued Apr. 23, 1991; hereby incorporated into the subject application by reference.

In an embodiment of the invention, cationic surfactants may optionally be used in the precursor liquid and foam cleansing composition of the present invention.

One class of cationic surfactants includes heterocyclic ammonium salts such as cetyl or stearyl pyridinium chloride, alkyl amidoethyl pyrrylinodium methyl sulfate, and lapyrium chloride.

Tetra alkyl ammonium salts are another useful class of cationic surfactants suitable for use. Examples include cetyl or stearyl trimethyl ammonium chloride or bromide; hydrogenated palm or tallow trimethylammonium halides; behenyl trimethyl ammonium halides or methyl sulfates; decyl isononyl dimethyl ammonium halides; ditallow (or distearyl) dimethyl ammonium halides, and behenyl dimethyl ammonium chloride.

Still other types of cationic surfactants that may be used are the various ethoxylated quaternary amines and ester quats. Examples include PEG-5 stearyl ammonium lactate (e.g., Genamin KSL manufactured by Clariant), PEG-2 coco ammonium chloride, PEG-15 hydrogenated tallow ammonium chloride, PEG 15 stearyl ammonium chloride, dipalmitoyl ethyl methyl ammonium chloride, dipalmitoyl hydroxyethyl methyl sulfate, and strearyl amidopropyl dimethylamine lactate.

Still other useful cationic surfactants suitable for use include quaternized hydrolysates of silk, wheat, and keratin proteins, and it is within the scope of the invention to use mixtures of the aforementioned cationic surfactants.

If used, cationic surfactants will make up no more than 1.0% by weight of the foam precursor liquid and foam cleansing composition. When present, they typically make up from 0.01 to 0.7%, and more typically, from 0.1 to 0.5% by weight of the foam precursor liquid and foam cleansing composition, including all ranges subsumed therein.

In an embodiment of this invention, the foam precursor liquid and foam cleansing composition will be substantially free of polymeric quaternary ammonium compounds (including salts of the same). In another embodiment, the foam precursor liquid and foam cleansing composition will comprise less than 0.1% by weight polymeric quaternary ammonium compounds. In yet another embodiment, the liquid and composition comprise less than 0.01% by weight polymeric quaternary ammonium compounds. In even another embodiment, the liquid and composition are free of polymeric quaternary ammonium compounds (i.e., 0.0%).

Water preferably makes up from 65 to 95% by weight of the liquid and composition, and most preferably, from 70 to 90% by weight water based on total weight of the liquid and composition, including all ranges subsumed therein.

The pH of the foam precursor liquid (and resulting foam) is typically from 4.5 to 10, and preferably, from 5 to 9, and most preferably, from 6 to 8.5, including all ranges subsumed therein. Adjusters suitable to modify/buffer the pH may be used. Such pH adjusters include triethylamine, NaOH, KOH, H₂SO₄, HCl, C₆ H₈ O₇ (i.e., citric acid) or mixtures thereof. The pH adjusters are added at amounts such that the resulting pH of the foam precursor liquid and foam composition are as defined and desired, from 4.5 to 10. The pH values may be assessed with commercial instrumentation such as a pH meter made commercially available from Thermo Scientific^{®}.

Optional skin benefit agents suitable for use in this invention are limited only to the extent that they are capable of being topically applied, and suitable to dissolve in the foam precursor liquid and foam cleansing composition at the desired pH.

Illustrative examples of the benefit agents suitable to include in the water portion of the liquid and composition are acids, like amino acids, such as arginine, valine or histidine. Additional water soluble benefit agents suitable for use include vitamin B_{2,} niacinamide (vitamin B₃), vitamin B₆, vitamin C, mixtures thereof or the like. Water soluble derivatives of such vitamins may also be employed. For instance, vitamin C derivatives such as ascorbyl tetraisopalmitate, magnesium ascorbyl phosphate and ascorbyl glycoside may be used alone or in combination with each other. Other water soluble benefit agents suitable for use include 4-ethyl resorcinol, extracts like sage, aloe vera, green tea, grapeseed, thyme, chamomile, yarrow, cucumber, liquorice, rosemary extract or mixtures thereof. Water soluble sunscreens like ensulizole may also be used. Total amount of optional water soluble benefit agents (including mixtures) when present in the invention may range from 0.0 to 10%, preferably from 0.001 to 8%, and most preferably, from 0.01 to 6% by weight, based on total weight of the foam precursor liquid and foam cleansing composition (as the case may be) and including all ranges subsumed therein.

It is also within the scope of the present invention to optionally include oil (i.e., non-water) soluble benefit agents. The only limitation with respect to such oil soluble benefit agents are that the same is suitable to provide a benefit to skin when topically applied.

Illustrative examples of the types of oil soluble benefit agents that may optionally be used in the liquid and composition of this invention include components like stearic acid, vitamins like Vitamin A, D, E and K (and their oil soluble derivatives), sunscreens like ethylhexylmethoxycinnamate, bis-ethyl hexyloxyphenol methoxyphenol triazine, 2-ethylhexyl-2-cyano-3,3-diphenyl-2-propanoic acid, drometrizole trisiloxane, 3,3,5-trimethyl cyclohexyl 2-hydroxybenzoate, 2-ethylhexyl-2-hydroxybenzoate or mixtures thereof. It may in particular be preferred that the concentrate, the precursor liquid or the foam composition comprises vitamin D.

Other optional oil soluble benefit agents suitable for use include resorcinols like 4-hexyl resorcinol, 4-phenylethyl resorcinol, 4-cyclopentyl resorcinol, 4-cyclohexyl resorcinol 4-isopropyl resorcinol or a mixture thereof. Also, 5-substituted resorcinols like 4-cyclohexyl-5-methylbenzene-1,3-diol, 4-isopropyl-5-methylbenzene-1,3-diol, mixtures thereof or the like may be used. The 5-substituted resorcinols, and their synthesis are described in commonly assigned U.S. Published Patent Application No. 2016/0000669A1.

Even other oil soluble actives suitable for use include omega-3 fatty acids, omega-6 fatty acids, climbazole, farnesol, ursolic acid, myristic acid, geranyl geraniol, oleyl betaine, cocoyl hydroxyethyl imidazoline, hexanoyl sphingosine, 12-hydroxystearic acid, petroselinic acid, conjugated linoleic acid, terpineol, thymol mixtures thereof or the like.

In an embodiment of the invention, the optional oil soluble benefit agent used is a retinoic acid precursor. In one embodiment of the invention, the retinoic acid precursor is retinol, retinal, retinyl propionate, retinyl palmitate, retinyl acetate or a mixture thereof. Retinyl propionate, retinyl palmitate and mixtures thereof are typically preferred.

Still another retinoic acid precursor suitable for use is hydroxyanasatil retinoate made commercially available under the name Retextra^{®} as supplied by Molecular Design International. The same may be used in a mixture with the oil soluble actives described herein.

When optional (i.e., 0.0 to 2% by weight) oil soluble active is used in the oil phase of the liquid and composition of the invention, it typically makes up from 0.001 to 1.5%, and in another embodiment, from 0.05 to 1.2%, and in yet another embodiment, from 0.1 to 0.5% by weight of the total weight of the liquid and composition, as the case may be, including all ranges subsumed therein.

Preservatives can desirably be incorporated into the foam precursor liquid and foam cleansing composition to protect against the growth of potentially harmful microorganisms. Cosmetic chemists are familiar with appropriate preservatives and routinely choose them to satisfy the preservative challenge test and to provide product stability. Suitable traditional preservatives for use include hydantoin derivatives and propionate salts. Particularly preferred preservatives are iodopropynyl butyl carbamate, phenoxyethanol, 1,2-octanediol, hydroxyacetophenone, ethylhexylglycerine, hexylene glycol, methyl paraben, propyl paraben, imidazolidinyl urea, sodium dehydroacetate, dimethyl-dimethyl (DMDM) hydantoin and benzyl alcohol and mixtures thereof. Other preservatives suitable for use include sodium dehydroacetate, chlorophenesin and decylene glycol. The preservatives should be selected having regard for the use of the composition and possible incompatibilities between the preservatives and other ingredients in the emulsion. Preservatives are preferably employed in amounts ranging from 0.01% to 2% by weight of the total weight of the composition, including all ranges subsumed therein. Also preferred is a preservative system with hydroxyacetophenone alone or in a mixture with other preservatives.

Thickening agents are optionally suitable for use in the foam precursor liquid and foam cleansing composition of the present invention. Particularly useful are the polysaccharides. Examples include fibers, starches, natural/synthetic gums and cellulosics. Representative of the starches are chemically modified starches such as sodium hydroxypropyl starch phosphate and aluminum starch octenylsuccinate. Tapioca starch is often preferred, as is maltodextrin. Suitable gums include xanthan, sclerotium, pectin, karaya, arabic, agar, guar (including Acacia senegal guar), carrageenan, alginate and combinations thereof. Suitable cellulosics include hydroxypropyl cellulose, hydroxypropyl methylcellulose, ethylcellulose, sodium carboxy methylcellulose (cellulose gum/carboxymethyl cellulose) and cellulose (e.g. cellulose microfibrils, cellulose nanocrystals or microcrystalline cellulose). Sources of cellulose microfibrils include secondary cell wall materials (e.g. wood pulp, cotton), bacterial cellulose, and primary cell wall materials. Preferably the source of primary cell wall material is selected from parenchymal tissue from fruits, roots, bulbs, tubers, seeds, leaves and combination thereof; more preferably is selected from citrus fruit, tomato fruit, peach fruit, pumpkin fruit, kiwi fruit, apple fruit, mango fruit, sugar beet, beet root, turnip, parsnip, maize, oat, wheat, peas and combinations thereof; and even more preferably is selected from citrus fruit, tomato fruit and combinations thereof. A most preferred source of primary cell wall material is parenchymal tissue from citrus fruit. Citrus fibers, such as those made available by Herbacel^{®} as AQ Plus can also be used as source for cellulose microfibrils. The cellulose sources can be surface modified by any of the known methods including those described in Colloidal Polymer Science, Kalia et al., "Nanofibrillated cellulose: surface modification and potential applications" (2014), Vol 292, Pages 5-31.

Synthetic polymers are yet another class of effective thickening agent. This category includes crosslinked polyacrylates such as the Carbomers, polyacrylamides such as Sepigel^{®} 305 and taurate copolymers such as Simulgel^{®} EG and Aristoflex^{®} AVC, the copolymers being identified by respective INCI nomenclature as Sodium Acrylate/Sodium Acryloyldimethyl Taurate and Acryloyl Dimethyltaurate/Vinyl Pyrrolidone Copolymer. Another preferred synthetic polymer suitable for thickening is an acrylate-based polymer made commercially available by Seppic and sold under the name Simulgel INS100. Calcium carbonate, fumed silica, and magnesium-aluminum-silicate may also be used.

The amounts of the thickening agent, when used, should preferably not increase the viscosity of the foam precursor liquid by more than 10% of such liquid without the thickening agent. Typical amounts may range from 0.001 to 5%, by weight of the liquid and composition. Maltodextrin, xanthan gum, and carboxymethyl cellulose are the often preferred thickening agents.

Fragrances, fixatives, chelators (like EDTA) salts (like NaCl) and exfoliants may optionally be included in the liquid and composition of the present invention. Each of these substances may range from about 0.03 to about 5%, preferably between 0.1 and 3% by weight of the total weight of the liquid and composition, including all ranges subsumed therein. To the extent the exfoliants are used, those selected should be of small enough particle size so that they do not impede the performance of the pump and actuator used to dispense the foam cleansing composition of this invention.

Conventional emulsifiers having an HLB of greater than 8 are often preferred. Illustrative examples include Tween, 40, 60, 80, polysorbate 20 and mixtures thereof. Typically emulsifiers for water continuous systems make up from 0.3 to 2.5% by weight of the liquid and composition.

Conventional humectants may optionally be employed as additives in the present invention to assist in moisturizing skin when such emulsions are topically applied. These are generally polyhydric alcohol type materials. Typical polyhydric alcohols include glycerol (i.e., glycerine or glycerin), propylene glycol, dipropylene glycol, polypropylene glycol (e.g., PPG-9), polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. Most preferred is glycerin, propylene glycol or a mixture thereof. The amount of humectant employed may range anywhere from 0.0 to 35% by weight of the total weight of the precursor liquid and foam composition. Often, humectant makes up from 0.0 to 20%, and preferably, from 0.001 to 15% by weight (most preferably, from 2 to 12% by weight) of the total weight of the precursor liquid and foam composition.

When making the foam precursor liquid of the present invention, the desired ingredients may be mixed with conventional apparatus under moderate shear atmospheric conditions, with temperature being from ambient to 90°C. In an embodiment of the invention, the foam cleansing composition has a compression force from 210 to 550. In still another embodiment the foam cleansing composition has a compression force of 215 to 300 mN, including all ranges subsumed therein. In even another embodiment of the invention, the cellulose ether used is first dissolved in water heated from 40 to 90°C and cooled to ambient temperature prior to mixing the resulting solution with additional ingredients.

The invention relates to a cleansing foam precursor liquid. In particular, the invention relates to the combination of the cleansing foam precursor liquid with a pump foam dispenser. The precursor liquid is filled in the pump foam dispenser. The packaging for the foam precursor liquid of this invention is typically a pump foam dispenser that is equipped with mesh having a pore size from 30 to 250 microns, and an operational peak force (force needed for full pump depression) from 15 to 50 Newtons whereby such dispenser is suitable to dispense the foam precursor liquid of this invention as foam cleansing composition that is 70 to 95% air, and further wherein the volume of foam dispensed from the dispenser is 5 to 15 times the volume of actual foam precursor liquid dispensed from the bottle of the pump foam dispenser.

Pump foam dispensers suitable for use with the foam precursor liquid of the present invention preferably have mesh with a pore size from 35 to 140 microns, and most preferably, from 45 to 135 microns; and an operational peak force from 18 to 45 Newtons, and preferably, from 20 to 40 Newtons; and the volume of foam dispensed from the dispenser is 6 to 12, and preferably, from 7 to 10 times the volume of actual foam precursor liquid dispensed from the bottle of the pump foam dispenser. Often preferred pump foam dispensers comprise a dual mesh system for foam precursor liquid to pass through prior to exiting an orifice for making foam cleansing composition. When a dual mesh system is used, the first mesh typically has a pore size from 60 to 250 microns, and preferably, from 70 to 180 microns, and most preferably, from 85 to 160 microns, and the second mesh typically has a pore size from 35 to 90 microns, and preferably, from 40 to 75 microns, and most preferably, from 37 to 65 microns. As to such dispensers that are suitable for use with the foam precursor liquid of this invention, the same are commercially available from suppliers like Albea and Rieke pump manufacturers.

The Examples provided are to facilitate an understanding of the invention. They are not intended to limit the scope of the claims.

### EXAMPLE I

The control formulations described in Table 1 were made by mixing the ingredients under moderate shear and atmospheric pressure and with temperature at about 45°C. Mixing continued until a homogeneous composition was obtained. The pH of the formulations was about 7.5.

**Table 1**

| **Control Liquid Formulations** | | |
|---|---|---|
| **Ingredient** | **Formula A** | **Formula B** |
| | **% Active in Formula** | **% Active in Formula** |
| Water | Balance | Balance |
| Chelator | 0.05 | 0.05 |
| Sodium Hydroxide | 0.00 | 0.05 |
| Sodium Lauroyl Glutamate | 6.40 | 8.00 |
| Stearic Acid | 0.31 | 0.31 |
| Citric Acid Anhydrous | 0.13 | 0.35 |
| Glycerin | 5.00 | 5.00 |
| PPG-9 | 0.50 | 0.50 |
| Sodium Lauroyl Glycinate | 3.18 | 3.98 |
| Cocamidopropyl Betaine | 2.85 | 3.56 |
| Preservative | 0.61 | 0.61 |
| Polysorbate 20 | 1.10 | 1.10 |
| Fragrance | 1.10 | 1.10 |
| **TOTAL** | **100.0** | **100.0** |

### EXAMPLE II

The foam precursor liquids depicted in Table 2 were made in a manner similar to the one described to make the controls of Example I except that cellulose ethers and conventional thickeners were added at 0.5% by weight and balanced with water. The liquids were discharged from an Albea foam dispenser having an exit/actuator and dual mesh, yielding a foam that was 75% air and having a foam volume that was ten (10) times the volume of the foam precursor liquid.

**Table 2**

| Foam Precursor Liquid | | | | | | |
|---|---|---|---|---|---|---|
| Cellulose ethers vs. conventional thickeners (0.5% inclusion) | DS | Methoxyl % by Wt. Substitution | MS | Hydroxypropyl % by Wt. Substitution | Compression Force (mN) | Pump Clog |
| Control (Formula A) | | | | | 182 | - |
| Methocel A | 1. 8 | 30 | 0.23 | 8.5 | - | Clogs Immediately |
| Methocel E | 1. 9 | 29 | 0.23 | 8.5 | 221 | None |
| Methocel E | 1. 9 | 29 | 0.1 3 | 5.0 | 248 | None |
| Methocel F | 1. 8 | 28 | 0.1 3 | 5.0 | 228 | None |
| Methocel F | 1. 8 | 28 | 0.2 1 | 8.1 | 279 | None |
| Methocel K | 1. 4 | 22 | | | 178 | |
| Merquat 3940 | | | | | 189 | |
| Merquat 740 | | | | | 185 | |
| Control (Formula B) | | | | | 196 | |
| Pemulen TR1 | | | | | 225 | Clogs over time |
| Synthalen W2000 | | | | | 238 | Clogs over time |
| Aculyn 33 | | | | | 231 | Clogs over time |
| Guar Hydroxypropyltrimoniu m Chloride (C14S) | | | | | | Clogs Immediately |
| | | | | | | |
| | | | | | | |

| | | | | | | |
|---|---|---|---|---|---|---|
| i) Methocel-Dow Chemical, hydroxypropyl methylcellulose; letter coincides with DS and MS, viscosity (samples with a Methocel E and F) in 2% water solution of 0.5 to 250 cps. ii) Merquat 3940-Lubrizol, ampholytic terpolymer; Merquat 740, polyquarternium-7. iii) Pemulen TR1-Lubrizol, C₁₀-C₃₀ alkyl acrylate. iv) Synthalen W2000-3V, anionic acrylic copolymer. v) Aculyn 33-Dow Chemical, anionic acrylic polymer emulsion. vi) Guar Hydroxypropyltrimonium chloride-Lamberti, Mn about 1.0 million | | | | | | |

The data in Table 2 surprisingly shows that when foam precursor liquid is made according to the present invention, foam and lather characteristics of the foam cleansing composition produced are superior in that the compression force exceeds 205 mN (an Albea dual mesh pump was used, first mesh 90 microns and second mesh 75 microns).

### EXAMPLE III

The foam precursor liquids in Table 3 below were made in a manner similar to the one used to make control liquids in Example I. Pumping to discharge liquid as foam composition was done as in Example 2. Surfactant percent means the total weight percent surfactant in the liquid in a weight percent ratio of about 2:1:1 glutamate/glycinate/betaine.

**TABLE 3**

| Pump | Average Peak |
|---|---|
| | Compression Force (mN) |
| 8% Surfactant (Control | 176 |
| 11 % Surfactant (Control) | 171 |
| 12.5% Surfactant (Control) | 179 |
| 14.15% Surfactant (Control) | 188 |
| 8% Surfactant + 0.5% Methocel E50 | 214 |
| 11 % Surfactant + 0.5% Methocel E50 | 210 |
| 14.15% Surfactant + 0.5% Methocel E50 | 232 |

The data in Table 3 shows that excellent foam and lather characteristics are obtained even when varying the surfactant levels in the foam precursor liquid. Liquids made consistent with the invention had a compression force of 210 or higher.

## Claims

1. A liquid concentrate composition for a cleansing foam precursor liquid, the concentrate composition comprising:
a) cellulose ether comprising hydroxypropyl methylcellulose, the hydroxypropyl methylcellulose having a degree of methoxy substitution (DS) from 1.77 to 1.94 and a hydroxypropyl molar substitution (MS) from 0.10 to 0.25;
b) from 5 to 70% by weight of anionic surfactant, and from 5 to 70% by weight amphoteric and/or zwitterionic surfactant, the anionic to amphoteric and/or zwitterionic surfactant at a weight ratio from 5:1 to 1:5; and
c) from 15 to 40% by weight water,
wherein the cellulose ether makes up from 0.25 to 5% by weight of the foam precursor liquid concentrate and when present at 2.0% by weight in water yields a solution having a viscosity from 0.5 to 250 cps, taken with a Discovery HR-2 Rheometer using sand blasted plates with a 100 micron gap and a shear rate of 5-15 s⁻¹ at 25 °C.

2. The concentrate composition according to claim 1 wherein the cellulose ether is hydroxypropyl methylcellulose.

3. The concentrate composition according to claim 1 or 2, wherein the liquid further comprises water soluble actives, oil soluble actives or both.

4. A cleansing foam precursor liquid filled in a pump foam dispenser, the composition comprising:
a) 0.25 to 5% by weight cellulose ether comprising hydroxypropyl methylcellulose, the hydroxypropyl methylcellulose having a degree of methoxy substitution (DS) from 1.77 to 1.94 and a hydroxypropyl molar substitution (MS) from 0.10 to 0.25;
b) from 2 to 35% by weight of anionic surfactant, and from 2 to 35% by weight amphoteric and/or zwitterionic surfactant, the anionic to amphoteric and/or zwitterionic surfactant at a weight ratio from 5:1 to 1:5; and
c) from 55 to 95.75% by weight water,
wherein the cellulose ether when present at 2.0% by weight in water yields a solution having a viscosity from 0.5 to 250 cps, and
wherein the pump foam dispenser preferably has a pore size from 30 to 250 microns, and wherein the composition has a viscosity of between 25 and 3000 cps,
and wherein viscosities are taken with a Discovery HR-2 Rheometer using sand blasted plates with a 100 micron gap and a shear rate of 5-15 s-1 at 25 °C.

5. The cleansing foam precursor liquid according to claim 4, wherein the precursor liquid comprises 0.001 to 8% water soluble active, 0.001 to 1.5% oil soluble active or both.

6. The cleansing foam precursor liquid according to any one of claims 4 or 5, wherein the precursor liquid comprises 3 to 16% by weight total weight surfactant.

7. The cleansing foam precursor liquid according to any one of claims 4 to 6, wherein the precursor liquid has a pH from 4.5 to 10.

8. The cleansing foam precursor liquid according to any one of the claims 4 to 7, wherein the dispenser has an operational peak force from 15 to 50 N.

9. The cleansing foam precursor liquid according to any one of the claims 4 to 8, wherein the dispenser is refillable.

10. The cleansing foam precursor liquid according to any one of the preceding claims, wherein the precursor liquid is substantially free of quaternary ammonium compounds, wherein "substantially free of" means less than 1.0% by weight.

## Patentansprüche

1. Flüssige Konzentratzusammensetzung für eine Reinigungsschaum-Vorläuferflüssigkeit, wobei die Konzentratzusammensetzung umfasst:
a) Celluloseether, der Hydroxypropylmethylcellulose umfasst, wobei die Hydroxypropylmethylcellulose einen Methoxy-Substitutionsgrad (DS) von 1,77 bis 1,94 und eine Hydroxypropyl-Molsubstitution (MS) von 0,10 bis 0,25 aufweist;
b) 5 bis 70 Gew.-% anionisches Tensid und 5 bis 70 Gew.-% amphoteres und/oder zwitterionisches Tensid, wobei das Gewichtsverhältnis von anionischem zu amphoterem und/oder zwitterionischem Tensid zwischen 5:1 und 1:5 liegt; und
c) 15 bis 40 Gew.-% Wasser,
wobei der Celluloseether 0,25 bis 5 Gew.-% des Konzentrats der Schaumvorläuferflüssigkeit ausmacht und bei einem Gehalt von 2,0 Gew.-% in Wasser eine Lösung einer Viskosität von 0,5 bis 250 cps ergibt, gemessen mit einem Discovery HR-2-Rheometer unter Verwendung sandgestrahlter Platten mit einer Spaltweite von 100 Mikrometern und einer Schergeschwindigkeit von 5-15 s⁻¹ bei 25°C.

2. Konzentratzusammensetzung nach Anspruch 1, wobei der Celluloseether Hydroxypropylmethylcellulose ist.

3. Konzentratzusammensetzung nach Anspruch 1 oder 2, wobei die Flüssigkeit ferner wasserlösliche Wirkstoffe, öllösliche Wirkstoffe oder beides umfasst.

4. Reinigungsschaum-Vorläuferflüssigkeit, abgefüllt in einem Pump-Schaumspender, wobei die Zusammensetzung umfasst:
a) 0,25 bis 5 Gew.-% Celluloseether, der Hydroxypropylmethylcellulose umfasst, wobei die Hydroxypropylmethylcellulose einen Methoxy-Substitutionsgrad (DS) von 1,77 bis 1,94 und eine Hydroxypropyl-Molsubstitution (MS) von 0,10 bis 0,25 aufweist;
b) 2 bis 35 Gew.-% anionisches Tensid und 2 bis 35 Gew.-% amphoteres und/oder zwitterionisches Tensid, wobei das Gewichtsverhältnis von anionischem zu amphoterem und/oder zwitterionischem Tensid zwischen 5:1 und 1:5 liegt; und
c) 55 bis 95,75 Gew.-% Wasser,
wobei der Celluloseether bei einem Gehalt von 2,0 Gew.-% in Wasser eine Lösung einer Viskosität von 0,5 bis 250 cps ergibt und
wobei der Pump-Schaumspender vorzugsweise eine Porengröße von 30 bis 250 Mikrometern aufweist und wobei die Zusammensetzung eine Viskosität zwischen 25 und 3000 cps aufweist, und
wobei die Viskositäten mit einem Discovery HR-2-Rheometer unter Verwendung sandgestrahlter Platten mit einer Spaltweite von 100 Mikrometern und einer Schergeschwindigkeit von 5-15 s⁻¹ bei 25°C gemessen werden.

5. Reinigungsschaum-Vorläuferflüssigkeit nach Anspruch 4, wobei die Vorläuferflüssigkeit 0,001 bis 8% wasserlöslichen Wirkstoff, 0,001 bis 1,5% öllöslichen Wirkstoff oder beides enthält.

6. Reinigungsschaum-Vorläuferflüssigkeit nach irgendeinem der Ansprüche 4 oder 5, wobei die Vorläuferflüssigkeit 3 bis 16 Gew.-% des Gesamtgewichts Tensid enthält.

7. Reinigungsschaum-Vorläuferflüssigkeit nach irgendeinem der Ansprüche 4 bis 6, wobei die Vorläuferflüssigkeit einen pH-Wert von 4,5 bis 10 aufweist.

8. Reinigungsschaum-Vorläuferflüssigkeit nach irgendeinem der Ansprüche 4 bis 7, wobei der Spender eine maximale Betätigungskraft von 15 bis 50 N aufweist.

9. Reinigungsschaum-Vorläuferflüssigkeit nach irgendeinem der Ansprüche 4 bis 8, wobei der Spender nachfüllbar ist.

10. Reinigungsschaum-Vorläuferflüssigkeit nach einem der vorhergehenden Ansprüche, wobei die Vorläuferflüssigkeit im Wesentlichen frei von quaternären Ammoniumverbindungen ist, wobei "im Wesentlichen frei von" weniger als 1,0 Gew.-% bedeutet.

## Revendications

1. Composition de concentré liquide pour un liquide précurseur de mousse nettoyante, la composition de concentré comprenant:
a) un éther de cellulose comprenant de l'hydroxypropyl-méthylcellulose, l'hydroxypropylméthylcellulose ayant un degré de substitution par méthoxy (DS) de 1,77 à 1,94 et une substitution molaire par hydroxypropyle (MS) de 0,10 à 0,25;
b) de 5 à 70 % en poids de tensioactif anionique, et de 5 à 70 % en poids de tensioactif amphotère et/ou zwitterionique, le tensioactif anionique à amphotère et/ou zwitterionique à un rapport en poids de 5:1 à 1:5; et
c) de 15 à 40 % en poids d'eau,
dans laquelle l'éther de cellulose constitue de 0,25 à 5 % en poids du concentré de liquide précurseur de mousse et, lorsqu'il est présent à raison de 2,0 % en poids dans l'eau, donne une solution ayant une viscosité de 0,5 à 250 cPs, mesurée avec un rhéomètre Discovery HR-2 utilisant des plateaux sablés avec un entrefer de 100 microns et une vitesse de cisaillement de 5 à 15 s⁻¹ à 25°C.

2. Composition de concentré selon la revendication 1 dans laquelle l'éther de cellulose est l'hydroxypropylméthylcellulose.

3. Composition de concentré selon la revendication 1 ou 2, dans laquelle le liquide comprend en outre des actifs hydrosolubles, des actifs liposolubles ou les deux.

4. Liquide précurseur de mousse nettoyante chargé dans un distributeur de mousse à pompe, la composition comprenant:
a) 0,25 à 5 % en poids d'éther de cellulose comprenant de l'hydroxypropylméthylcellulose, l'hydroxypropylméthylcellulose ayant un degré de substitution par méthoxy (DS) de 1,77 à 1,94 et une substitution molaire par hydroxypropyle (MS) de 0,10 à 0,25;
b) de 2 à 35 % en poids de tensioactif anionique et de 2 à 35 % en poids de tensioactif amphotère et/ou zwitterionique, le tensioactif anionique à amphotère et/ou zwitterionique à un rapport en poids de 5:1 à 1:5; et
c) de 55 à 95,75 % en poids d'eau,
dans lequel l'éther de cellulose, lorsqu'il est présent à raison de 2,0 % en poids dans l'eau, donne une solution ayant une viscosité de 0,5 à 250 cPs, et
dans lequel le distributeur de mousse à pompe a de préférence une taille de pores de 30 à 250 microns, et dans lequel la composition a une viscosité entre 25 et 3000 cPs,
et dans lequel les viscosités sont mesurées avec un rhéomètre Discovery HR-2 utilisant des plateaux sablés avec un entrefer de 100 microns et une vitesse de cisaillement de 5 à 15 s⁻¹ à 25°C.

5. Liquide précurseur de mousse nettoyante selon la revendication 4, dans lequel le liquide précurseur comprend 0,001 à 8 % d'actif hydrosoluble, 0,001 à 1,5 % d'actif liposoluble ou les deux.

6. Liquide précurseur de mousse nettoyante selon l'une quelconque des revendications 4 ou 5, dans lequel le liquide précurseur comprend 3 à 16 % en poids de tensioactif par rapport au poids total.

7. Liquide précurseur de mousse nettoyante selon l'une quelconque des revendications 4 à 6, dans lequel le liquide précurseur a un pH de 4,5 à 10.

8. Liquide précurseur de mousse nettoyante selon l'une quelconque des revendications 4 à 7, dans lequel le distributeur a une force maximale opérationnelle de 15 à 50 N.

9. Liquide précurseur de mousse nettoyante selon l'une quelconque des revendications 4 à 8, dans lequel le distributeur est rechargeable.

10. Liquide précurseur de mousse nettoyante selon l'une quelconque des revendications précédentes, dans lequel le liquide précurseur est sensiblement exempt de composés d'ammonium quaternaire, dans lequel « sensiblement exempt de » signifie moins de 1,0 % en poids.
